# EUROPEAN PATENT APPLICATION

(11) **EP 4 674 834 A1**
(43) Date of publication of application: **07.01.2026**
(21) Application number: 24764030.3
(22) Date of filing: 01.03.2024
(51) Int. Cl.: C07C 25/18, C07C 43/225, C07C 205/12

(54) **HETEROPOLYOXOTUNGSTATE COMPOUND, SOLVATE THEREOF, OR MIXTURE OF COMPOUND AND SOLVATE THEREOF, AND METHOD FOR PRODUCING COMPOUND, SOLVATE THEREOF, OR MIXTURE OF COMPOUND AND SOLVATE THEREOF**

(30) Priority: 02.03.2023 JP 2023032119
(71) Applicant: TOKYO OHKA KOGYO CO., LTD., Kawasaki-shi, Kanagawa 211 0012 (JP)
(72) Inventor: INARI, Takatoshi, Kawasaki-shi, Kanagawa 211-0012 (JP); NODA, Kunihiro, Kawasaki-shi, Kanagawa 211-0012 (JP); KUBO, Keisuke, Kawasaki-shi, Kanagawa 211-0012 (JP); NISHIZAWA, Akito, Kawasaki-shi, Kanagawa 211-0012 (JP); KIMURA, Kenta, Kawasaki-shi, Kanagawa 211-0012 (JP); KINOSHITA, Yohei, Kawasaki-shi, Kanagawa 211-0012 (JP); WAKIYA, Kazumasa, Kawasaki-shi, Kanagawa 211-0012 (JP)
(74) Representative: Plasseraud IP
(86) International application number: PCT/JP2024/007709
(87) International publication number: WO 2024/181553

(57) **Abstract**

To provide a heteropolyoxotungstate compound represented by the general formula (I) or a solvate thereof or a mixture of them, and a method for producing them.

(A^{m+})_{x/m}(Bⁿ⁺)_{y/n}(C^{-(x+y)}) (I)

## Description

### Technical Field

The present invention relates to a heteropolyoxotungstate compound or a solvate thereof or a mixture thereof and a method for producing them.

### Background Art

A polyoxometalate (POM) is an anionic metal oxide cluster and has a structure in which an early transition metal with an electron configuration of d⁰ or d¹ (for example, Mo (hexavalent or pentavalent), W (hexavalent or pentavalent), V (pentavalent), Nb (pentavalent), Ta (pentavalent), or the like) is oxygen-cross-linked.

Various structures can be adopted by incorporating various elements into the anionic metal oxide cluster of POM. Depending on the combination of constituent elements of POM, the structure thereof, and the like, physicochemical properties, such as oxidation-reduction ability, light absorption ability, and acidity, can be changed.

For example, Patent Literature 1 describes that a POM containing polyoxomolybdic acid and a compound with a pyridinium skeleton has a high photoluminescence quantum yield.

Polyoxotungstic acid (POT), which is one type of POM, includes an isopolyoxotungstic acid composed of an oxygen acid containing tungsten and a heteropolyoxotungstic acid composed of an oxygen acid containing tungsten and one or more heteroatoms other than tungsten. The heteroatom constituting the heteropolyoxotungstic acid can be selected from a large number of Groups I to VIII elements, such as Si, Ge, and P, and POMs with different physicochemical properties can be formed by changing the combination, constituent ratio, and the like thereof.

Patent Literature 2 discloses a photochromic article containing a complex of a polyoxometalate anion, such as phosphotungstic acid, and a counter cation, such as an ammonium cation.

### Citation List

### Patent Literature

PTL 1: U.S. Patent No. 11192908
PTL 2: U.S. Patent No. 10711182

### Summary of Invention

### Technical Problem

In view of the above circumstances, an object of the present invention is to provide a novel heteropolyoxotungstate compound or a solvate thereof or a mixture thereof and a method for producing them.

### Solution to Problem

As a result of intensive studies to solve the above problems, the present inventors have succeeded in synthesizing a heteropolyoxotungstate compound represented by the general formula (I) or a solvate thereof or a mixture thereof and have completed the present invention by finding a method for efficiently synthesizing a heteropolyoxotungstate compound represented by the general formula (I) or a solvate thereof or a mixture thereof.

More specifically, the present invention relates to the following inventions.
<1> A heteropolyoxotungstate compound represented by the general formula (I) or a solvate thereof:

   (A^{m+})_{x/m}(Bⁿ⁺)_{y/n}(C^{-(x+y)}) (I)

   wherein
   A^{m+} each independently denotes H⁺, a metal ion, or an ammonium ion;
   Bⁿ⁺ each independently denotes an iodonium cation;
   C^{-(x+y)} denotes a heteropolyoxotungstic acid; and
   m denotes an integer in the range of 1 to 5, n denotes an integer of 1 or 2, x and x/m denote an integer, and y and y/n denote a natural number.
<2> The heteropolyoxotungstate compound or the solvate thereof according to <1>, wherein the heteropolyoxotungstic acid is a heteropolyoxotungstic acid represented by the general formula (II-1) or (II-2), or a heteropolyoxotungstic acid represented as a deficient species thereof.

   [XW₁₂O₄₀]^{-z1} (II-1)

   [X₂W₁₈O₆₂]^{-z2} (II-2)

   [In the formulae,
   X each independently denotes B, Al, Si^{IV}, Ge^{IV}, P^{V}, As^{V}, V^{V}, Cr^{III}, Fe^{III}, Co^{III}, Co^{II} , Cu^{II}, Cu^{I}, Zn, Se^{VI}, or S^{VI}, and
   z1 and z2 denote a natural number.]
<3> The heteropolyoxotungstate compound or the solvate thereof according to <2>, wherein the heteropolyoxotungstic acid represented by (II-1) is [PW₁₂O₄₀]³⁻, [SiW₁₂O₄₀]⁴⁻, [BW₁₂O₄₀]⁵⁻, or [SW₁₂O₄₀]²⁻.
<4> The heteropolyoxotungstate compound or the solvate thereof according to <2>, wherein the heteropolyoxotungstic acid represented by (II-2) is [P₂W₁₈O₆₂]⁶⁻, [Si₂W₁₈O₆₂]⁸⁻, or [S₂W₁₈O₆₂]⁴⁻.
<5> A heteropolyoxotungstate mixture containing two or more different heteropolyoxotungstate compounds or solvates thereof according to <1>.
<6> A heteropolyoxotungstate mixture containing two or more different heteropolyoxotungstate compounds or solvates thereof according to <1>, wherein the heteropolyoxotungstic acid is silicotungstic acid of Keggin type or Dawson type or a deficient species thereof, and
   the mole ratio I:W of I to W in XRF analysis or XPS analysis is 0.5 to 10:9 to 18.
<7> A heteropolyoxotungstate mixture containing two or more different heteropolyoxotungstate compounds or solvates thereof according to <1>, wherein the heteropolyoxotungstic acid is phosphotungstic acid of Keggin type or Dawson type or a deficient species thereof, and
   the mole ratio I:W of I to W in XRF analysis or XPS analysis is 0.5 to 6:9 to 18.
<8> The heteropolyoxotungstate compound or a solvate thereof according to <1>, wherein the iodonium cation is an iodonium cation represented by the general formula (III):

   R^{3A}-I⁺-R^{3B} (III)

   wherein
   R^{3A} and R^{3B} each independently denote an optionally substituted C₁₋₁₈ hydrocarbyl group, an optionally substituted 3- to 18-membered non-aromatic heterocyclic group, or an optionally substituted 5- to 18-membered aromatic heterocyclic group;
   R^{3A} and R^{3B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with an iodine atom in the formula (III).
<9> The heteropolyoxotungstate compound or the solvate thereof according to <1>, wherein the iodonium cation is an iodonium cation represented by the general formula (IV-1):

   Ar^{4A}-I⁺-Ar^{4B} (IV-I)

   wherein
   Ar^{4A} and Ar^{4B} each independently denote an optionally substituted C₆₋₁₈ aryl group or 5- to 18-membered heterocyclic group,
   in the optionally substituted C₆₋₁₈ aryl group or 5- to 18-membered heterocyclic group, at least part of its hydrogen atoms may be substituted by a halogen atom, a hydroxy group, a thiol group, a nitro group, a cyano group, a carboxy group, an amino group, a sulfo group, or at least part of its hydrogen atoms may be substituted by a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, each of which may be substituted by a halogen atom, a hydroxy group, a thiol group, a nitro group, a cyano group, a carboxy group, an amino group, a sulfo group, or a group in which a hydrogen atom of a carboxy group or a hydroxy group is substituted by an acid-dissociable group,
   a divalent carbon atom at any position excluding the terminals of the substituent may be replaced by -O-, -C(=O)-, -COO-, -OCO-, -CONH-, -NHCO-, -S-, or -SO₂-(provided that adjacent divalent carbon atoms are not replaced at the same time),
   Ar^{4A} and Ar^{4B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with an iodine atom in the formula (IV-1); or
   an iodonium cation represented by the general formula (IV-2): 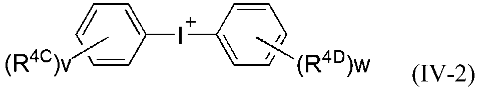 wherein
   R^{4C} and R^{4D} each independently denote a halogen atom, a hydroxy group, a thiol group, a nitro group, a cyano group, a carboxy group, an amino group, a sulfo group, or each independently denote a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of its hydrogen atoms may be substituted by a halogen atom, a hydroxy group, a thiol group, a nitro group, a cyano group, a carboxy group, an amino group, or a sulfo group,
   a divalent carbon atom at any position excluding the terminals of these may be replaced by -O-, -C(=O)-, -COO-, -OCO-, -CONH-, -NHCO-, -S-, or -SO₂- (provided that adjacent divalent carbon atoms are not replaced at the same time),
   v and w each independently denote an integer in the range of 0 to 5, and when v to w denote an integer of 2 or more, R^{4A} and R^{4D} may be the same or different.
<10> The heteropolyoxotungstate compound or the solvate thereof according to <9>, wherein in an iodonium cation represented by the general formula (IV-1), Ar^{4B} denotes an iodonium cation represented by the general formula (IV-3):

   Ar^{4A}-I⁺-Ar^{4B} (IV-1)

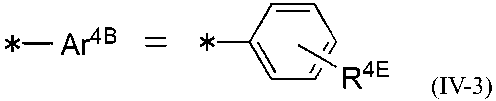

   wherein
   R^{4E} denotes a halogen atom, a nitro group, a C₁₋₄ haloalkyl group, or an optionally substituted branched C₃₋₁₀ alkyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group, and
   when R^{4E} denotes a halogen atom, a nitro group, or a C₁₋₄ haloalkyl group, Ar^{4A} and Ar^{4B} are different from each other.
<11> The heteropolyoxotungstate compound or the solvate thereof according to <9>, wherein in an iodonium cation represented by the general formula (IV-1), Ar^{4B} denotes an iodonium cation represented by the general formula (IV-4):

   Ar^{4A}-I⁺-Ar^{4B} (IV-1)

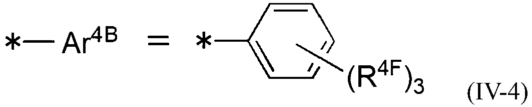

   wherein
   R^{4F} each independently denotes an optionally substituted C₁₋₁₂ hydrocarbyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group.
<12> A method for producing a heteropolyoxotungstate compound represented by the general formula (I) or a solvate thereof, the method comprising:
   reacting a compound represented by the general formula (V-1) with a compound represented by the general formula (V-2) to perform salt exchange;

   (A^{m+})_{x/m}(Bⁿ⁺)_{y/n}(C^{-(x+y)}) (I)

   (A^{m+})_{x/m}(A'^{m'+})_{y/m'}(C^{-(x+y)}) (V-I)

   (Bⁿ⁺)_{y/n}(Y⁻)_{y} (V-2)

   wherein
   A^{m+} each independently denotes H⁺, a metal ion, or an ammonium ion;
   A'^{m'+} each independently denotes a metal ion or an ammonium ion;
   Bⁿ⁺ each independently denotes an iodonium cation;
   C^{-(x+y)} denotes a heteropolyoxotungstic acid;
   Y⁻ denotes a monovalent counter anion; and
   m and m' denote an integer in the range of 1 to 5, n denotes an integer of 1 or 2, x and x/m denote an integer, and y/m', y, and y/n denote a natural number.
<13> A method for producing a heteropolyoxotungstate compound represented by the general formula (I) or a solvate thereof, the method comprising:
   reacting a compound represented by the general formula (V-1) with a compound represented by the general formula (V-2) to perform salt exchange; and
   removing a compound represented by the general formula (V-3) from the resulting compounds represented by the general formulae (I) and (V-3);

      (A^{m+})_{x/m}(Bⁿ⁺)_{y/n}(C^{-(x+y)}) (I)

      (A^{m+})_{x/m}(A'^{m'+})_{y/m'}(C^{-(x+y)}) (V-I)

      (Bⁿ⁺)_{y/n}(Y⁻)_{y} (V-2)

      (A'^{m'+})_{y/m'}(Y⁻)_{y} (V-3)

      wherein
      A^{m+} each independently denotes H⁺, a metal ion, or an ammonium ion;
      A'^{m'+} each independently denotes a metal ion or an ammonium ion;
      Bⁿ⁺ each independently denotes an iodonium cation;
      C^{-(x+y)} denotes a heteropolyoxotungstic acid;
      Y⁻ denotes a monovalent counter anion; and
      m and m' denote an integer in the range of 1 to 5, n denotes an integer of 1 or 2, x and x/m denote an integer, and y/m', y, and y/n denote a natural number.
<14> A method for producing a heteropolyoxotungstate compound represented by the general formula (I) or a solvate thereof, the method comprising:
   ion-exchanging A'^{m'+} of a compound represented by the general formula (V-1) with H⁺ using an ion-exchange resin; and
   reacting the resulting compound represented by the general formula (V-4) with a compound represented by the general formula (V-2);

      (A^{m+})_{x/m}(Bⁿ⁺)_{y/n}(C^{-(x+y)}) (I)

      (A⁻⁺)_{x/m}(A'^{m'+})_{y/m}(C^{-(x+y}) (V-I)

      (A^{m+})_{x/m}(H⁺)_{y}(C^{-(x+y)}) (V-4)

      (Bⁿ⁺)_{y/n}(Y⁻)_{y} (V-2)

      wherein
      A^{m+} each independently denotes H⁺, a metal ion, or an ammonium ion;
      A'^{m'+} each independently denotes a metal ion or an ammonium ion;
      Bⁿ⁺ each independently denotes an iodonium cation;
      C^{-(x+y)} denotes a heteropolyoxotungstic acid;
      Y⁻ denotes a monovalent counter anion; and
   m and m' denote an integer in the range of 1 to 5, n denotes an integer of 1 or 2, x and x/m denote an integer, and y/m', y, and y/n denote a natural number.

### Advantageous Effects of Invention

The present invention can provide a novel heteropolyoxotungstate compound or a solvate thereof or a mixture thereof and a method for producing them.

### Description of Embodiments

Preferred embodiments of the present invention will be described in detail below. However, the present invention is not limited to the following embodiments.

### [1. Heteropolyoxotungstate Compound or Solvate Thereof or Mixture of Them]

A heteropolyoxotungstate compound according to an embodiment of the present invention is represented by the following general formula (I).

(A^{m+})_{x/m}(Bⁿ⁺)_{y/n}(C^{-(x+y)}) (I)

[In the formula,
A^{m+} each independently denotes H⁺, a metal ion, or an ammonium ion,
Bⁿ⁺ each independently denotes an iodonium cation,
C^{-(x+y)} denotes a heteropolyoxotungstic acid, and
m denotes an integer in the range of 1 to 5, n denotes an integer of 1 or 2, x and x/m denote an integer, and y and y/n denote a natural number.]
A^{m+} is hereinafter also referred to as "another counter cation".

"Heteropolyoxotungstic acid", "iodonium cation", "another counter cation", "compound or solvate or mixture of them", and "solvate" will be sequentially described.

### [1-1. Heteropolyoxotungstic Acid]

A heteropolyoxotungstate compound according to the present embodiment is represented by the following general formula (I).

(A^{m+})_{x/m}(Bⁿ⁺)_{y/n}(C^{-(x+y)}) (I)

C^{-(x+y)} denotes a heteropolyoxotungstic acid, and
x denotes an integer, and y denotes a natural number.

The heteropolyoxotungstic acid according to the present embodiment is not particularly limited, and a known and commonly used heteropolyoxotungstic acid can be used. As the heteropolyoxotungstic acid, for example, a Keggin type, a Dawson type, or a deficient species thereof, as well as an Anderson type, a lacunary Keggin type, a substituted Keggin type, and a Strandberg type are known, and any of these may be used.

It should be noted that the heteropolyoxotungstic acid may include all isomers, such as geometric isomers, optical isomers, rotational isomers, stereoisomers, and tautomers, which are structurally present, and isomer mixtures, and is not limited to the description of convenient chemical formulae.

It is known that Keggin-type heteropolyoxotungstic acids ([XW₁₂O₄₀]^{-z1}: X each independently denotes B, Al, Si^{IV}, Ge^{IV}, P^{V}, As^{V}, V^{V}, Cr^{III}, Fe^{III}, Co^{III}, Co^{II}, Cu^{II}, Cu^{I}, Zn, Se^{VI}, or S^{VI}, and z1 denotes a natural number) include α-Keggin type, β-Keggin type, γ-Keggin type, and the like. The chemical formula [XW₁₂O₄₀]^{-z1} includes the isomers or mixtures of these isomers.

Likewise, it is known that Dawson-type heteropolyoxotungstic acids ([X₂W₁₈O₆₂]^{-z2}: X each independently denotes B, Al, Si^{IV}, Ge^{IV}, P^{V}, As^{V}, V^{V}, Cr^{III}, Fe^{III}, Co^{III}, Co^{II}, Cu^{II}, Cu^{I}, Zn, Se^{VI}, or S^{VI}, and z2 denotes a natural number) include an α-Dawson type, a β-Dawson type, a γ-Dawson type, and the like. The chemical formula [X₂W₁₈O₆₂]^{-z2} includes the isomers or mixtures of these isomers.

From the perspective of high thermal stability and stability in an oxidizing atmosphere, the heteropolyoxotungstic acid according to the present embodiment is preferably of the Keggin type, of the Dawson type, or a deficient species thereof.

The Keggin-type heteropolyoxotungstic acid is, for example, [PW₁₂O₄₀]³⁻, [SiW₁₂O₄₀]⁴⁻, [BW₁₂O₄₀]⁵⁻, [SW₁₂O₄₀]²⁻, or the like. The Dawson-type heteropolyoxotungstic acid is, for example, [P₂W₁₈O₆₂]⁶⁻, [Si₂W₁₈O₆₂]⁸⁻, [S₂W₁₈O₆₂]⁴⁻, or the like. The Keggin-type or Dawson-type deficient heteropolyoxotungstic acid is, for example, [PW₉O₃₄]⁹⁻, [PW₁₀O₃₆]⁷⁻, [PW₁₁O₃₉]⁷⁻, [SiW₉O₃₄]¹⁰⁻, [SiW₁₀O₃₆]⁸⁻, [SiW₁₁O₃₉]⁸⁻, [P₂W₁₇O₆₁]¹⁰⁻, or the like.

Among these, the heteropolyoxotungstic acid according to the present embodiment is preferably [PW₁₂O₄₀]³⁻, [SiW₁₂O₄₀]⁴⁻, [BW₁₂O₄₀]⁵⁻, [SW₁₂O₄₀]²⁻, [P₂W₁₈O₆₂]⁶⁻, [Si₂Wi₈O₆₂]⁸⁻, [S₂W₁₈O₆₂]⁴⁻, more preferably [PW₁₂O₄₀]³⁻, [SiW₁₂O₄₀]⁴⁻, [BW₁₂O₄₀]⁵⁻, or [SW₁₂O₄₀]²⁻.

### [1-2. iodonium cation]

A heteropolyoxotungstate compound according to the present embodiment is represented by the following general formula (I):

(A^{m+})_{x/m}(Bⁿ⁺)_{y/n}(C^{-(x+y)}) (I)

Bⁿ⁺ each independently denotes an iodonium cation; and
n denotes an integer of 1 or 2, and y and y/n denote a natural number.

Meanings of terms, symbols, and the like used in the present description will be described below, and embodiments of the present invention will be described in more detail below.

The term "halogen atom", as used herein, refers to a fluorine atom, a chlorine atom, a bromine atom, or an iodine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -S-, -SO-, or -SO₂-(provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -COO-, -OCO-, - CONH-, -NHCO-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -COO-, -OCO-, -CONH-, -NHCO-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methyl vinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -COO-, -OCO-, -CONH-, - NHCO-, -S-, or -SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbonyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bomylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₆₋₁₈ aryl group", as used herein, refers to an aromatic hydrocarbon ring group with 6 to 18 carbon atoms and may be a monocyclic, polycyclic, or fused ring.

The "C₆₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₆₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "5- to 18-membered aromatic heterocyclic group", as used herein, refers to a monocyclic, polycyclic, or condensed 5- to 18-membered aromatic heterocyclic group containing one or more heteroatoms selected from the group consisting of a nitrogen atom, an oxygen atom, and a sulfur atom.

The "5- to 18-membered aromatic heterocyclic group" is, for example, but not limited to, a pyrrolyl group, a furyl group, a thienyl group, an imidazolyl group, a triazolyl group, a tetrazolyl group, a pyrazolyl group, an oxazolyl group, an isoxazolyl group, a thiazolyl group, an isothiazolyl group, furazanyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthyridinyl group, a quinoxalinyl group, a cinnolinyl group, a quinazolinyl group, a phthalazinyl group, an imidazopyridyl group, an imidazothiazolyl group, an imidazooxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -COO-, -OCO-, -CONH-, - NHCO-, -S-, or -SO₂- (provided that adjacent divalent carbon atoms are not replaced at the same time).

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₆₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group".

The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonylamino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The phrase "optionally substituted", as used herein, is not particularly limited as long as it is chemically acceptable and has the advantages of the present invention.

The "substituent" is, for example, (1) a halogen atom, (2) a hydroxy group, (3) a thiol group, (4) a nitro group, (5) a cyano group, (6) a carboxy group, (7) an amino group, (8) a sulfo group, or (9) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of its hydrogen atoms may be substituted by (1) to (9), and a divalent carbon atom at any position excluding the terminals of the substituent may be replaced by -O-, -C(=O)-, -COO-, -OCO-, -CONH-, -NHCO-, -S-, or - SO₂- (provided that adjacent divalent carbon atoms are not replaced at the same time).

### [1-2-1. Iodonium Cation (III)]

An iodonium cation according to the present embodiment can be an iodonium cation represented by the following general formula (III).

R^{3A}-I⁺--R^{3B} (III)

In an iodonium cation represented by the general formula (III),
R^{3A} and R^{3B} each independently denote an optionally substituted C₁₋₁₈ hydrocarbyl group, an optionally substituted 3- to 18-membered non-aromatic heterocyclic group, or an optionally substituted 5- to 18-membered aromatic heterocyclic group, and
R^{3A} and R^{3B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with the iodine atom in the formula (V-1).

In an iodonium cation represented by the general formula (III), for example, any one of R^{3A} and R^{3B} may be an optionally substituted C₂₋₁₈ alkynyl group among optionally substituted C₁₋₁₈ hydrocarbyl groups.

Examples of the case where any one of R^{3A} and R^{3B} denotes an optionally substituted C₁₋₁₈ alkynyl group include the following iodonium cation.

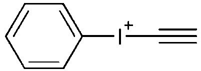

When R^{3A} and R^{3B} denote optionally substituted C₆₋₁₈ aryl groups, they may be linked to each other directly by a single bond or via a divalent linking group -O-, -S-, - C(=O)-, -S(=O)-, -S(=O)₂-, -C(=O)O-, or a C₁₋₃ alkylene group to form a ring together with the iodine atom in the formula (III).

Examples of the case where a ring is formed include the following iodonium cations and the like.

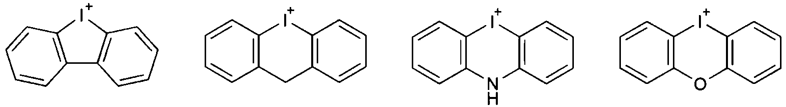

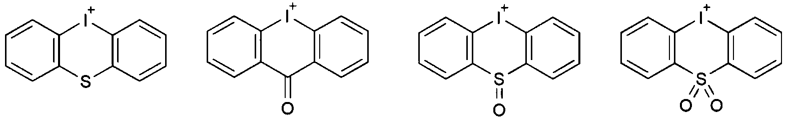

### [1-2-2. Iodonium Cation (IV)]

The iodonium cation according to the present embodiment may be an iodonium cation represented by (IV-1) or (IV-2).

Ar^{4A}-I⁺-Ar^{4B} (IV-1)

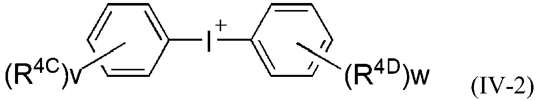

### [1-2-2-1. Iodonium Cation (IV-1)]

In an iodonium cation represented by the general formula (IV-1),

Ar^{4A}-I⁺-Ar^{4B} (IV-I)

Ar^{4A} and Ar^{4B} each independently denote an optionally substituted C₆₋₁₈ aryl group or 5- to 18-membered heterocyclic group,
in the optionally substituted C₆₋₁₈ aryl group or 5- to 18-membered heterocyclic group, at least part of the hydrogen atoms may be substituted by a halogen atom, a hydroxy group, a thiol group, a nitro group, a cyano group, a carboxy group, an amino group, or a sulfo group, or the optionally substituted C₆₋₁₈ aryl group or 5- to 18-membered heterocyclic group may be substituted by a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of the hydrogen atoms may be substituted by a halogen atom, a hydroxy group, a thiol group, a nitro group, a cyano group, a carboxy group, an amino group, or a sulfo group,
a divalent carbon atom at any position excluding the terminals of the substituent may be replaced by -O-, -C(=O)-, -COO-, -OCO-, -CONH-, -NHCO-, -S-, or -SO₂-(provided that adjacent divalent carbon atoms are not replaced at the same time), and
Ar^{4A} and Ar^{4B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with the iodine atom in the formula (IV-1).

In an iodonium cation represented by the general formula (IV-1), for example, any one or more of Ar^{4A} and Ar^{4B} may be an optionally substituted 5- to 18-membered heterocyclic group.

The iodonium cation is, for example, one of the following iodonium cations.

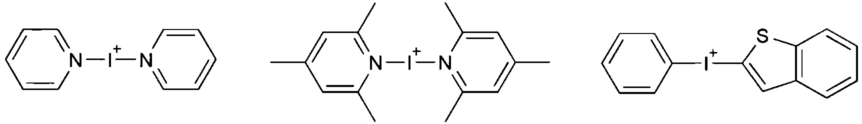

In an iodonium cation represented by the general formula (IV-1), Ar^{4A} and Ar^{4B} may be the same or different. Preferably, by making Ar^{4A} and Ar^{4B} different from each other, an iodonium cation represented by the general formula (IV-1) can be made asymmetric, and the crystallinity of the iodonium cation can therefore be lowered to improve the solubility.

### [1-2-2-2. Iodonium Cation (IV-2)]

In an iodonium cation represented by the general formula (IV-2), 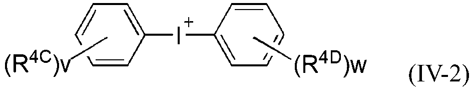
R^{4C} and R^{4D} each independently denote a halogen atom, a hydroxy group, a thiol group, a nitro group, a cyano group, a carboxy group, an amino group, or a sulfo group, or each independently denote a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of its hydrogen atoms may be substituted by a halogen atom, a hydroxy group, a thiol group, a nitro group, a cyano group, a carboxy group, an amino group, or a sulfo group,
a divalent carbon atom at any position excluding the terminals of these may be replaced by -O-, -C(=O)-, -COO-, -OCO-, -CONH-, -NHCO-, -S-, or -SO₂- (provided that adjacent divalent carbon atoms are not replaced at the same time), and
v and w each denote an integer in the range of 0 to 5, and when v and w denote an integer of 2 or more, R^{4C} and R^{4D} may be the same or different.

In an iodonium cation represented by the general formula (IV-2), (R^{4C})ᵥ and (R^{4D})_{w} may be the same or different. Preferably, by making (R^{4C})ᵥ and (R^{4D})_{w} different from each other, an iodonium cation represented by the general formula (IV-2) can be made asymmetric, and the crystallinity of the iodonium cation can therefore be lowered to improve the solubility.

In an iodonium cation represented by the general formula (IV-2), for example, R^{4C} and R^{4D} may denote, as an electron-donating group or an electron-withdrawing group, a halogen atom, a hydroxy group, a nitro group, a cyano group, an optionally substituted C₁₋₄ haloalkyl group, an optionally substituted C₁₋₁₈ hydrocarbyloxy group, an optionally substituted C₁₋₁₈ hydrocarbyloxycarbonyl group, an optionally substituted C₁₋₁₈ hydrocarbylcarbonyloxy group, or an optionally substituted C₁₋₁₈ hydrocarbylthio group.

The iodonium cation is, for example, one of the following iodonium cations.

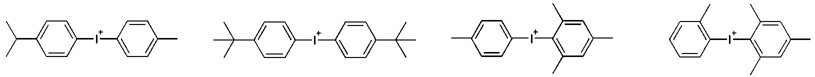

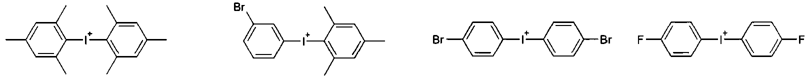

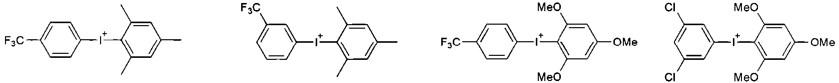

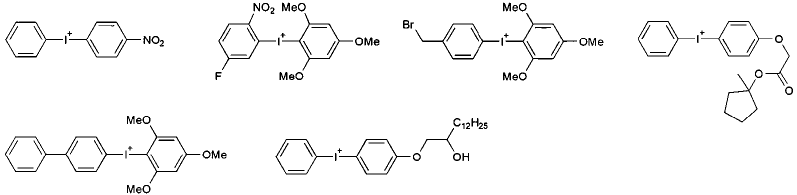

### [1-2-2-3. Iodonium Cation (IV-3)]

In another embodiment, in an iodonium cation represented by the following general formula (IV-1), Ar^{4B} may be an iodonium cation represented by the following general formula (IV-3).

Ar^{4A}-I⁺-Ar^{4B} (IV-1)

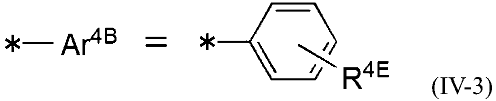

In the general formula (IV-3),
R^{4E} denotes a halogen atom, a nitro group, a C₁₋₄ haloalkyl group, or an optionally substituted branched C₃₋₁₀ alkyl group (for example, an i-propyl group, an i-butyl group, a sec-butyl group, a t-butyl group, a sec-pentyl group, a t-pentyl group, a neopentyl group, a 1-methylbutyl group, a 2-methylbutyl group, a 1,1-dimethylpropyl group, or a 1,2-dimethylpropyl group), a C₁₋₁₂ hydrocarbyloxy group, a C₁₋₁₂ hydrocarbylcarbonyl group, a C₁₋₁₂ hydrocarbylcarbonyloxy group, a C₁₋₁₂ hydrocarbyloxycarbonyl group, or a C₁₋₁₂ hydrocarbyloxycarbonyloxy group, and
when R^{4E} denotes a halogen atom, a nitro group, or a C₁₋₄ haloalkyl group, Ar^{4A} and Ar^{4B} are different from each other.

In the general formula (IV-3), from the perspective of reducing the crystallinity of the iodonium cation and improving the solubility, R^{4E} denotes a halogen atom, a nitro group, a C₁₋₄ haloalkyl group, an optionally substituted C₁₋₈ alkoxy group, or a branched C₃₋₈ alkyl group, and when R^{4E} denotes a halogen atom, a nitro group, or a C₁₋₄ haloalkyl group, Ar^{4A} and Ar^{4B} are preferably different asymmetric iodonium cations, more preferably, R^{4E} denotes a fluorine atom, a chlorine atom, a nitro group, or a C₁₋₄ haloalkyl group, and Ar^{4A} and Ar^{4B} are different asymmetric iodonium cations.

### [1-2-2-4. Iodonium Cation (IV-4)]

In another embodiment, in an iodonium cation represented by the following general formula (IV-1), Ar^{4B} may be an iodonium cation represented by the following general formula (IV-4).

Ar^{4A}-I⁺-Ar^{4B} (IV-1)

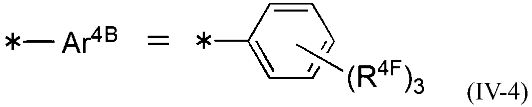

In the general formula (IV-4),
R^{4F} each independently denotes an optionally substituted C₁₋₁₂ hydrocarbyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group.

In the general formula (IV-4), from the perspective of improving the stability and solubility of the iodonium cation, R^{4F} preferably each independently denotes an optionally substituted C₁₋₁₀ alkyl group or C₁₋₁₀ alkoxy group, more preferably a C₁₋₈ alkyl group or a C₁₋₈ alkoxy group, still more preferably a C₁₋₄ alkyl group or a C₁₋₄ alkoxy group.

### [1-2-3. Specific Examples of Iodonium Cation]

Specific examples of the iodonium cations represented by the general formulae (III) to (IV-2) include an ethynyl(phenyl)iodonium cation, a bis(pyridine)iodonium cation, a bis(2,4,6-trimethylpyridine)iodonium cation, a diphenyliodonium cation, a bis(4-(t-butyl)phenyl)iodonium cation, a (2-carboxyphenyl)(phenyl)iodonium cation, a (4-nitrophenyl)(phenyl)iodonium cation, a (3-(trifluoromethyl)phenyl)(2,4,6-trimethylphenyl)iodonium cation, a bis(4-fluorophenyl)iodonium cation, a (4-(bromomethyl)phenyl)(2,4,6-trimethoxyphenyl)iodonium cation, a 4-biphenylyl(2,4,6-trimethoxyphenyl)iodonium cation, a bis(2,4,6-trimethylphenyl)iodonium cation, a 4-isopropyl-4'-methyldiphenyliodonium cation, a (4-(trifluoromethyl)phenyl)(2,4,6-trimethylphenyl)iodonium cation, a ((4-trifluoromethyl)phenyl)(2,4,6-trimethoxyphenyl)iodonium cation, a (5-fluoro-2-nitrophenyl)(2,4,6-trimethoxyphenyl)iodonium cation, a (3-bromophenyl)(mesityl)iodonium cation, a bis(4-bromophenyl)iodonium cation, a (3,5-dichlorophenyl)(2,4,6-trimethoxyphenyl)iodonium cation, a (4-methylphenyl)(2,4,6-trimethylphenyl)iodonium cation, a (3-methylphenyl)(2,4,6-trimethylphenyl)iodonium cation, a (2-methylphenyl)(2,4,6-trimethylphenyl)iodonium cation, a phenyl(2,4,6-trimethoxyphenyl)iodonium cation, and the like.

### [1-3. Other Counter Cations]

A heteropolyoxotungstate compound according to the present embodiment is represented by the following general formula (I):

(A^{m+})_{x/m}(Bⁿ⁺)_{y/n}(C-^{(x+y)}) (I)

A^{m+} each independently denotes H⁺, a metal ion, or an ammonium ion; and
m denotes an integer in the range of 1 to 5, and x and x/m denote an integer.

A metal ion that can be used as A^{m+} is, for example, but not limited to, Li⁺, Na⁺, K⁺, Rb⁺, Cs⁺, Be²⁺, Mg²⁺, Ca²⁺, Sr²⁺, Ba²⁺, Co³⁺, Bi³⁺, Zr⁴⁺, Hf⁴⁺, Bi⁵⁺, or the like. These metal ions may be used alone or in combination.

An ammonium cation that can be used as A ^{m+} is, for example, but not limited to, NH⁴⁺, a quaternary ammonium cation, or the like.

The quaternary ammonium cation can be represented by (R)₄N⁺. Each R is independent and may be the same or different. R is, for example, but not limited to, an optionally substituted C₁₋₁₈ hydrocarbyl group or the like. R preferably denotes an optionally substituted C₁₋₁₂ hydrocarbyl group, more preferably an optionally substituted C₁₋₈ hydrocarbyl group.

From the perspective of the stability and reactivity of the heteropolyoxotungstate, A^{m+} is preferably Li⁺, Na⁺, K⁺, Rb⁺, Cs⁺, Mg²⁺, Ca²⁺, Sr²⁺, Ba²⁺, Co³⁺, NH⁴⁺, or a quaternary ammonium cation, more preferably Na⁺, K⁺, Rb⁺, Cs⁺, Co³⁺, NH⁴⁺, or a quaternary ammonium cation, still more preferably NH⁴⁺ or a quaternary ammonium cation.

In a heteropolyoxotungstate compound or a compound represented by the following formula (I), the ratio of x/m to y/n is not particularly limited and can be appropriately determined depending on the stability, reactivity, use, and the like of the heteropolyoxotungstate compound.

(A^{m+})_{x/m}(Bⁿ⁺)_{y/n}(C^{-(x+y)}) (I)

For example, when x + y is 3 and m and n are 1, from the perspective of achieving both the reactivity and the stability of a heteropolyoxotungstate compound, x:y is preferably 0 to 2:1 to 3, more preferably 0 to 2:2 to 3, still more preferably 0 to 1:2 to 3. In the above case, preferably, x denotes an integer in the range of 0 to 2, and y denotes an integer in the range of 1 to 3, preferably, x denotes an integer in the range of 0 to 2, and y denotes an integer of 2 or 3, more preferably x denotes an integer of 0 or 1, and y denotes an integer of 2 or 3.

For example, when x + y is 4 and m and n are 1, from the perspective of achieving both the reactivity and the stability of a heteropolyoxotungstate compound, x:y is preferably 0 to 3:1 to 4, more preferably 0 to 2:1 to 4, still more preferably 0 to 2:2 to 4. In the above case, preferably, x denotes an integer in the range of 0 to 3, and y denotes an integer in the range of 1 to 4, more preferably, x denotes an integer in the range of 0 to 2, and y denotes an integer in the range of 1 to 4, still more preferably, x denotes an integer in the range of 0 to 2, and y denotes an integer in the range of 2 to 4.

### [1-4. Compound or Solvate or Mixture of Them]

A heteropolyoxotungstate mixture according to the present embodiment may contain two or more different heteropolyoxotungstate compounds represented by the following general formula (I) or solvates thereof.

(A^{m+})_{x/m}(Bⁿ⁺)_{y/n}(C^{-(x+y)}) (I)

A^{m+} each independently denotes H⁺, a metal ion, or an ammonium ion,
Bⁿ⁺ each independently denotes an iodonium cation,
C^{-(x+y)} denotes a heteropolyoxotungstic acid, and
m denotes an integer in the range of 1 to 5, n denotes an integer of 1 or 2, x and x/m denote an integer of 0 or more, and y and y/n denote a natural number.

In the heteropolyoxotungstate mixture, the heteropolyoxotungstic acid represented by C^{-(x+y)} can be, for example, but is not limited to, [PW₁₂O₄₀]³⁻, [SiW₁₂O₄₀]⁴⁻, [BW₁₂O₄₀]⁵⁻, [SW₁₂O₄₀]²⁻, [P₂W₁₈O₆₂]⁶⁻, [Si₂W₁₈O₆₂]⁸⁻, [S₂W₁₈O₆₂]⁴⁻, [PW₉O₃₄]⁹⁻, [PW₁₀O₃₆]⁷⁻, [PW₁₁O₃₉]⁷⁻, [SiW₉O₃₄]¹⁰⁻, [SiW₁₀O₃₆]⁸⁻, [SiW₁₁O₃₉]⁸⁻, [P₂W₁₇O₆₁]¹⁰⁻, or the like.

In the heteropolyoxotungstate mixture, the number of Bⁿ⁺s (iodonium cations) bonded to one heteropolyoxotungstic acid is not particularly limited, and for example, two or more different heteropolyoxotungstate compounds in which two or more of Bⁿ⁺s selected from an integer from 1 to 10 are bonded to one heteropolyoxotungstic acid may be contained. Specific examples thereof may include one Bⁿ⁺, two Bⁿ⁺s, and three Bⁿ⁺s bonded to one heteropolyoxotungstic acid.

Furthermore, in two or more different heteropolyoxotungstate compounds represented by the general formula (I) or solvates thereof, when the heteropolyoxotungstic acids represented by C^{-(x+y)} are common to each other, a plurality of heteropolyoxotungstate compounds having different numbers of A^{m+}s and Bⁿ⁺s bonded thereto or solvates thereof are included.

The heteropolyoxotungstate mixture in this case is represented by the following general formula (I').

(A1⁺)_{xym}(Bⁿ⁺)_{y'/n}(C^{-(x'+y')}) (I)

[In the formula,
A^{m+} each independently denotes H⁺, a metal ion, or an ammonium ion,
Bⁿ⁺ each independently denotes an iodonium cation,
C^{-(x'+y')} denotes a heteropolyoxotungstic acid, and
m denotes an integer in the range of 1 to 5, n denotes an integer of 1 or 2, x' and x'/m denote a real number of 0 or more, y' and y'/n denote a real number of more than 0, and x' + y' is a natural number.]

The number of Bⁿ⁺s (iodonium cations) bonded to one heteropolyoxotungstic acid can be changed by appropriately adjusting the blend ratio of the heteropolyoxotungstic acid and the raw material of the iodonium cation used in the production of the heteropolyoxotungstate compound, the solvate, or the mixture of them.

When an iodonium cation is used as Bⁿ⁺ in a heteropolyoxotungstate compound or a mixture, the elemental composition of I and W contained in the heteropolyoxotungstate compound or the mixture can be determined by XRF or XPS analysis.

The XRF analysis can be a known and commonly used method, and, for example, the mole ratio of S to W in the heteropolyoxotungstate compound or the mixture can be determined by a calibration curve method, SQX analysis using a fundamental parameter (FP) method, or the like.

The XPS analysis can be a known and commonly used method, and, for example, the mole ratio of S to W in the heteropolyoxotungstate compound or the mixture can be determined by determining Atomic% of each element based on the peak areas of I3d and W4f.

In a heteropolyoxotungstate mixture, when silicotungstic acid of the Keggin-type or the Dawson-type or a deficient species thereof is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 10:9 to 18, more preferably 1 to 10:9 to 18, still more preferably 2 to 8:10 to 18.

In a heteropolyoxotungstate mixture, when a Keggin-type or Dawson-type silicotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 8:12 to 18, more preferably 1 to 8:12 to 18, still more preferably 2 to 8:12 to 18.

In a heteropolyoxotungstate mixture, when a Keggin-type silicotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 4:12, more preferably 1 to 4:12, still more preferably 2.5 to 4:12.

In a heteropolyoxotungstate mixture, when a Dawson-type silicotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 8:18, more preferably 1 to 8:18, still more preferably 2 to 8:18.

In a heteropolyoxotungstate mixture, when phosphotungstic acid of the Keggin-type or the Dawson-type or a deficient species thereof is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 6:9 to 18, more preferably 1 to 6:9 to 18, still more preferably 2 to 6:10 to 18.

In a heteropolyoxotungstate mixture, when a Keggin-type or Dawson-type phosphotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 6:12 to 18, more preferably 1 to 6:12 to 18, still more preferably 2 to 6:12 to 18.

In a heteropolyoxotungstate mixture, when a Keggin-type phosphotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 3:12, more preferably 1 to 3:12, still more preferably 1.5 to 3:12.

In a heteropolyoxotungstate mixture, when a Dawson-type phosphotungstic acid is used as a heteropolyoxotungstic acid, and an iodonium cation is used as Bⁿ⁺, the mole ratio I:W of I (iodine) to W (tungsten) in the heteropolyoxotungstate mixture determined by XRF or XPS analysis is preferably 0.5 to 6:18, more preferably 1 to 6:18, still more preferably 2 to 6:18.

### [1-5. Solvate]

It is generally known that when a heteropolyoxotungstate is crystallized, the heteropolyoxotungstate takes in a solvent used for crystallization and forms a solvate.

The solvate of the heteropolyoxotungstate compound or the mixture according to the present embodiment is, for example, but not limited to, an alcohol solvate, such as a methanol solvate, an ethanol solvate, or an isopropanol solvate; a hydrate, such as a monohydrate, a dihydrate, a trihydrate, or a tetrahydrate; or the like.

### [2. Method for Producing Heteropolyoxotungstate Compound or Solvate Thereof]

A method for producing a heteropolyoxotungstate compound or a solvate thereof according to another embodiment of the present invention is, for example, but not limited to, a salt exchange method, an ion exchange method, or the like.

### [2-1. Salt Exchange Method]

A method for producing a heteropolyoxotungstate compound represented by the general formula (I) or a solvate thereof includes:
reacting a compound represented by the general formula (V-1) with a compound represented by the general formula (V-2) to perform salt exchange.

(A^{m+})_{x/m}(Bⁿ⁺)_{y/n}(C^{-(x+y)}) (I)

(A^{m+})_{x/m}(A'^{m'+})_{y/m'}(C^{-(x+y)}) (V1)

(Bⁿ⁺)_{y/n}(Y⁻)_{y} (V-2)

[wherein
A^{m+} each independently denotes H⁺, a metal ion, or an ammonium ion;
A'^{m'+} each independently denotes a metal ion or an ammonium ion;
Bⁿ⁺ each independently denotes an iodonium cation;
C^{-(x+y)} denotes a heteropolyoxotungstic acid;
Y⁻ denotes a monovalent counter anion; and
m and m' denote an integer in the range of 1 to 5, n denotes an integer of 1 or 2, x and x/m denote an integer, and y/m', y, and y/n denote a natural number.]

In the general formula (V-2), for example, when n is 1, the formula is expressed as (B⁺)_{y}(Y⁻)_{y}, which means y(B⁺·Y⁻).

A method for producing a heteropolyoxotungstate compound represented by the general formula (I) or a solvate thereof according to another embodiment may include:
reacting a compound represented by the general formula (V-1) with a compound represented by the general formula (V-2) to perform salt exchange; and
removing a compound represented by the general formula (V-3) from the resulting compounds represented by the general formulae (I) and (V-3),

   (A⁻⁺)_{x/m}(Bⁿ⁺)_{y/n}(C^{-(x+y)}) (I)

   (A^{m+})_{x/m}(A'^{m'+})_{y/m'}(C^{-(x+y)}) (V-I)

   (Bⁿ⁺)_{y/n}(Y⁻)_{y} (V-2)

   (A'^{m'+})_{y/m'}(Y⁻)_{y} (V-3)

   [wherein
   A^{m+} each independently denotes H⁺, a metal ion, or an ammonium ion;
   A'^{m'+} each independently denotes a metal ion or an ammonium ion;
   Bⁿ⁺ each independently denotes an iodonium cation;
   C^{-(x+y)} denotes a heteropolyoxotungstic acid;
   Y⁻ denotes a monovalent counter anion; and
   m and m' denote an integer in the range of 1 to 5, n denotes an integer of 1 or 2, x and x/m denote an integer, and y/m', y, and y/n denote a natural number.]

In the general formula (V-3), for example, when m' is 1, the formula is expressed as (A'⁺)_{y}(Y⁻)_{y}, which means y(A'⁺·Y⁻).

### <Compound Represented by General Formula (V-1)>

In a compound represented by the general formula (V-1), which is one of the raw materials in a method for producing a heteropolyoxotungstate compound or a solvate thereof according to the present embodiment,

(A^{m+})_{x/m}(A'^{m'+})_{y/m'}(C^{-(x+y)}) (V-1)

A^{m+} each independently denotes H⁺, a metal ion, or an ammonium ion, and
A'^{m'+} each independently denotes a metal ion or an ammonium ion.

From the perspective of promoting the salt exchange reaction, A'^{m'+} is preferably H⁺, Li⁺, Na⁺, K⁺, Rb⁺, Cs⁺, Be²⁺, Mg²⁺, Ca²⁺, Sr²⁺, Ba²⁺, Co³⁺, Bi³⁺, Zr⁴⁺, Hf⁴⁺, Bi⁵⁺, NH⁴⁺, a quaternary ammonium cation, or a combination thereof, more preferably H⁺, Na⁺, K⁺, Mg²⁺, Ca²⁺, NH⁴⁺, a quaternary ammonium cation, or a combination thereof, still more preferably H⁺, Na⁺, K⁺, NH⁴⁺, a quaternary ammonium cation, or a combination thereof.

A compound represented by the general formula (V-1) can be synthesized by a known synthesis method. A commercial product of a compound represented by the general formula (V-1) is, for example, phosphotungstic acid (H₃[PW₁₂O₄₀]·nH₂O) manufactured by Nippon Inorganic Colour & Chemical Co., Ltd., Fujifilm Wako Pure Chemical Corporation, or the like, silicotungstic acid (H₄[SiW₁₂O₄₀]·26H₂O) manufactured by Nippon Inorganic Colour & Chemical Co., Ltd., or the like.

### <Compound Represented by General Formula (V-2)>

In a compound represented by the general formula (V-2), which is one of the raw materials in a method for producing a heteropolyoxotungstate compound or a solvate thereof according to the present embodiment,

(Bⁿ⁺)_{y/n}(Y⁻)_{y} (V-2)

Y⁻ denotes a monovalent counter anion.

From the perspective of promoting the salt exchange reaction, Y⁻ is preferably Br⁻, Cl⁻, I⁻, OH⁻, CF₃SO₃⁻, C₂F₅SO₃⁻, C₃F₇SO₃⁻, C₄F₉SO₃⁻, p-CH₃(C₆H₄)SO₃⁻, ClSO₃⁻, ClO₄⁻, (C₆F₅)₄B-, BF₄⁻, PF₆⁻, SbF₆⁻, AlCl₄⁻, BiF₆⁻, or AsF₆⁻, more preferably Br⁻, Cl⁻, I⁻, OH⁻, CF₃SO₃⁻, C₄F₉SO₃⁻, BF₄⁻, PF₆⁻, or SbF₆⁻, still more preferably Br⁻, Cl⁻, CF₃SO₃⁻, BF₄⁻, PF₆⁻, or SbF₆⁻.

A commercial product of a compound represented by the general formula (V-2) is, for example, tributylsulfonium iodide, (thiodi-4,1-phenylene)bis(diphenylsulfonium)bis(hexafluorophosphate), 4-isopropyl-4'-methyldiphenyliodonium tetrakis(pentafluorophenyl)borate, (4-(bromomethyl)phenyl)(2,4,6-trimethoxyphenyl)iodonium p-toluenesulfonate, 4-biphenylyl(2,4,6-trimethoxyphenyl)iodonium trifluoromethanesulfonate, bis(4-t-butylphenyl)iodonium hexafluorophosphate, bis(2,4,6-trimethylphenyl)iodonium trifluoromethanesulfonate, bis(2,4,6-trimethylpyridine)iodonium hexafluorophosphate, bis(4-fluorophenyl)iodonium trifluoromethanesulfonate, ethynyl(phenyl)iodonium tetrafluoroborate, (4-(trifluoromethyl)phenyl)(2,4,6-trimethoxyphenyl)iodonium p-toluenesulfonate, (4-(trifluoromethyl)phenyl)(2,4,6-trimethylphenyl)iodonium trifluoromethanesulfonate, diphenyliodonium chloride, diphenyliodonium bromide, diphenyliodonium hexafluorophosphate, or diphenyliodonium trifluoromethanesulfonate each manufactured by Tokyo Chemical Industry Co., Ltd., or the like.

### <Blend Mole Ratio of Compound Represented by General Formula (V-1) to Compound Represented by General Formula (V-2)>

In a reaction of a compound represented by the general formula (V-1) and a compound represented by the general formula (V-2), the blend mole ratio of the compound represented by the general formula (V-1) to the compound represented by the general formula (V-2) is not particularly limited and can be appropriately determined according to the mole ratio of A^{m+} to Bⁿ⁺ contained in the target heteropolyoxotungstate or a solvate thereof.

The blend mole ratio of a compound represented by the general formula (V-1) to a compound represented by the general formula (V-2), the compound represented by the general formula (V-1):the compound represented by the general formula (V-2), is preferably 1 to 3:1 to 20, more preferably 1 to 3:1 to 18, still more preferably 2:1 to 15.

### <Reaction between Compound Represented by General Formula (V-1) and Compound Represented by General Formula (V-2)>

The reaction conditions of a compound represented by the general formula (V-1) and a compound represented by the general formula (V-2) are not particularly limited and can be known and commonly used reaction conditions, and the type of solvent, the reaction temperature, the reaction time, the pressure, and the like can be appropriately determined.

The type of solvent is, for example, but not limited to, water, methanol, ethanol, 1,4-dioxane, acetonitrile, 1-propanol, 2-propanol, ethyl acetate, acetonitrile, acetone, dichloromethane, chloroform, dimethylformamide, diethyl ether, or the like. These solvents may be used alone or in combination.

The reaction temperature is, for example, but not limited to, room temperature or a high temperature.

The reaction time is not particularly limited and is preferably 1 minute to 24 hours, more preferably 1 minute to 5 hours.

The pressure is not particularly limited and may be atmospheric pressure or a high pressure.

### <Step of Removing Compound Represented by General Formula (V-3)>

A method of removing a compound represented by the general formula (V-3) is, for example, but not limited to, a method of removing a compound represented by the general formula (V-3) formed as a by-product by extraction into an aqueous layer, a method of precipitation and filtration as an inorganic salt, a method of crystallization and filtration of a target compound represented by the general formula (I) or a solvate thereof, or the like.

(A'^{m'+})_{y/m'}(Y⁻)_{y} (V-3)

(A^{m+})_{x/m}(Bⁿ⁺)_{y/n}(C^{-(x+y)}) (I)

The method of crystallization and filtration of a target compound represented by the general formula (I) or a solvate thereof is, for example, but not limited to, a method of heating and dissolving the target compound in a solvent and then precipitating crystals by slow cooling, a method of combining a poor solvent and a good solvent for crystallization, or the like.

The poor solvent can be appropriately determined for the target compound and is, for example, diethyl ether, dipropyl ether, ethyl acetate, butyl acetate, hexane, toluene, 2-propanol, ethanol, methanol, or the like.

The good solvent can be appropriately determined for the target compound and is, for example, water, methanol, ethanol, 1,4-dioxane, dimethylformamide, chloroform, dichloromethane, or the like.

### [2-2. Ion Exchange Method]

A method for producing a heteropolyoxotungstate compound represented by the general formula (I) or a solvate thereof according to another embodiment includes:
ion-exchanging A'^{m'+} of a compound represented by the general formula (V-1) with H⁺ using an ion-exchange resin; and
reacting a compound represented by the general formula (V-4) produced by the above step with a compound represented by the general formula (V-2).

   (A^{m+})_{x/m}(Bⁿ⁺)_{y/n}(C^{-(x+y)}) (I)

   (A^{m+})_{x/m}(A'^{m'+})_{y/m'}(C^{-(x+y)}) (V-1)

   (A^{m+})_{x/m}(H⁺)_{y}(C^{-(x+y)}) (V-4)

   (Bⁿ⁺)_{y/n}(Y⁻)_{y} (V-2)

   [wherein
   A^{m+} each independently denotes H⁺, a metal ion, or an ammonium ion;
   A'^{m'+} each independently denotes a metal ion or an ammonium ion;
   Bⁿ⁺ each independently denotes an iodonium cation;
   C^{-(x+y)} denotes a heteropolyoxotungstic acid;
   Y⁻ denotes a monovalent counter anion; and
   m and m' denote an integer in the range of 1 to 5, n denotes an integer of 1 or 2, x and x/m denote an integer, and y/m', y, and y/n denote a natural number.]

### <Ion-Exchange Resin>

The ion-exchange resin is not particularly limited as long as A'^{m'+} of a compound represented by the general formula (V-1) can be ion-exchanged with H⁺ and can be a known and commonly used ion-exchange resin.

The ion-exchange resin is, for example, but not limited to, a strongly acidic cation-exchange resin, a weakly acidic cation-exchange resin, or the like.

Examples of commercial products of the ion-exchange resin include Amberlite (TM) IR-200CT, HPR1200 H, HPR1024 H, HPR1006NNC H, and Dowex (TM) 50W each manufactured by Dow Chemical; ORLITE (TM) DS-1 and DS-4 manufactured by Organo Corporation; and the like.

A method of ion-exchanging A'^{m'+} of a compound represented by the general formula (V-1) with H⁺ using an ion-exchange resin can be a known and commonly used method.

The method is, for example, but not limited to, a method of filling a column with an ion-exchange resin, then passing a solution, such as water or an organic solvent, containing a compound represented by the general formula (V-1) through the column to bring the compound represented by the general formula (V-1) and the ion-exchange resin into contact with each other, and performing elution with an eluting solvent, a method of mixing an ion-exchange resin with a compound represented by the general formula (V-1) in a beaker and then removing the ion-exchange resin by filtration, or the like.

The eluting solvent that can be used to pass the solution containing a compound represented by the general formula (V-1) through a column filled with an ion-exchange resin is, for example, but not limited to, Milli-Q water, pure water (ion-exchanged water, demineralized water), methanol, ethanol, or the like.

The space velocity (SV, h⁻¹) at which a compound represented by the general formula (V-1) is brought into contact using a column filled with an ion-exchange resin is not particularly limited as long as A'^{m'+} of the compound represented by the general formula (V-1) can be ion-exchanged with H⁺. The SV preferably ranges from 0.1 to 100, more preferably 0.5 to 50, still more preferably 1 to 30. The SV is a unit indicating how many times as much liquid as the resin volume is passed per hour.

### <Reaction with Compound Represented by General Formula (V-2)>

In a reaction of a compound represented by the general formula (V-4) produced by ion-exchanging A'^{m'+} of a compound represented by the general formula (V-1) with H⁺ and a compound represented by the general formula (V-2), the reaction conditions, the blend ratio of both, and the like can be appropriately determined within the range of the reaction conditions, the blend ratio, and the like similar to those in the salt exchange method.

(A^{m+})_{x/m}(H⁺)_{y}(C^{-(x+y)}) (V-4)

(Bⁿ⁺)_{y/n}(Y⁻)_{y} (V-2)

### <Step of Removing By-Product>

A reaction between a compound represented by the general formula (V-4) a nd a compound represented by the general formula (V-2) yields a heteropolyoxotun gstate compound represented by the general formula (I) and y(H⁺·Y⁻) as a by-prod uct. A method of removing the by-product can be the same as the method of re moving a compound represented by the general formula (V-3) in the salt exchange method.

### EXAMPLES

The present invention will be more specifically described below with reference to Examples, but the present invention is not limited to these Examples. In the following synthesis examples, when there is a discrepancy between the compound name and the chemical formula, the chemical formula takes precedence.

### [Synthesis Example 1: tetrakis(bis(4-tert-butylphenyl)iodonium)silicotungstate (M-1)]

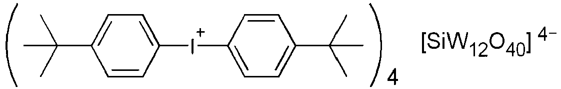

### <Salt Exchange Method>

36.76 g of bis(4-tert-butylphenyl)iodonium chloride was dissolved in 50 g of cyclohexanone, 41.06 g of silicotungstic acid (H₄[SiW₁₂O₄₀]·26H₂O) was added thereto, and the reaction liquid was stirred at room temperature for 2 hours. A powder produced by filtering the reaction liquid was dried under reduced pressure at room temperature for 18 hours. The dried powder was crystallized at room temperature using dichloromethane and acetonitrile to produce a target compound.
¹H-NMR (400 MHz, DMSO-D6): δ (ppm) = 8.19 (d, 16H), 7.51 (d, 16H), 1.22 (s, 72H). ¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### <Ion Exchange Method>

60.41 g of sodium silicotungstate was dissolved in 240 g of ion-exchanged water to prepare aqueous sodium silicotungstate. A column with an inner diameter of 30 mm was filled with 60 cm³ of a strongly acidic cation-exchange resin ORLITE (TM) DS-1 (manufactured by Organo Corporation). The aqueous sodium silicotungstate was passed through the column at a rate in the range of approximately 1 to 15 cm³/min, and ion-exchanged water was then passed through the column at the same rate. Among the eluates, an eluate with an acidic pH was collected.

23.00 g of bis(4-tert-butylphenyl)iodonium chloride was added to the collected eluate, and the reaction liquid was stirred at room temperature for 30 minutes. A white powder produced by filtering the reaction liquid was dried under reduced pressure at room temperature for 18 hours. The dried powder was crystallized at room temperature using dichloromethane and acetonitrile to produce a target compound.

### [Synthesis Example 2: tris(bis(4-tert-butylphenyl)iodonium)phosphotungstate (M-2)]

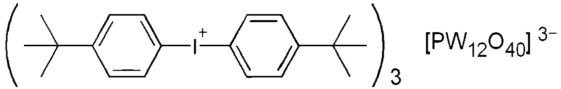

A target compound was produced in the same manner as in the salt exchange method of Synthesis Example 1 except that, as raw material compounds, 36.76 g of bis(4-tert-butylphenyl)iodonium chloride was changed to 27.57 g, and 41.97 g of phosphotungstic acid (H₃[PW₁₂O₄₀]·30H₂O) was used instead of silicotungstic acid.
¹H-NMR (400 MHz, DMSO-D6): δ (ppm) = 8.19 (d, 12H), 7.51 (d, 12H), 1.22 (s, 54H).
¹⁸³W-NMR (20.84 MHz, DMSO-D6): δ (ppm) = -86.7 (s, 12W).
ESI-MS: NEGATIVE m/z 959 (median) ([PW₁₂O₄₀]³⁻)
XPS analysis I:W (the mole ratio of I to W) = 3:12

### [Synthesis Example 3: tetrakis(dimesityliodonium)silicotungstate (M-3)]

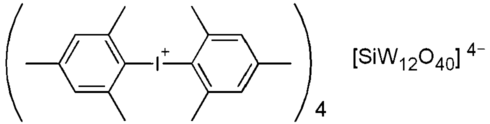

A target compound was produced in the same manner as in the salt exchange method of Synthesis Example 1 except that 34.36 g of dimesityliodonium chloride was used instead of 36.76 g of bis(4-tert-butylphenyl)iodonium chloride as a raw material compound.
¹H-NMR (400 MHz, DMSO-D6): δ (ppm) = 2.29 (s, 24H), 2.47 (s, 48H), 7.19 (s, 16H). ¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 4:tetrakis((4-nitrophenyl)(phenyl)iodonium)silicotungstate (M-4)]

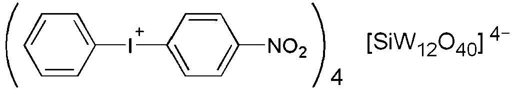

A target compound was produced in the same manner as in the salt exchange method of Synthesis Example 1 except that 31.00 g of (4-nitrophenyl)(phenyl)iodonium chloride was used instead of 36.76 g of bis(4-tert-butylphenyl)iodonium chloride as a raw material compound.
¹H-NMR (400 MHz, DMSO-D6): δ (ppm) = 7.30-7.51 (m, 28H), 8.20 (d, 8H). ¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 5: tetrakis(mesityl(3-(trifluoromethyl)phenyl)iodonium)silicotungstate (M-5)]

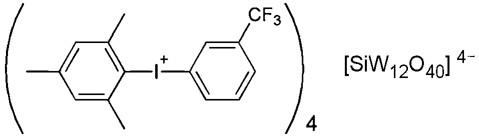

A target compound was produced in the same manner as in the salt exchange method of Synthesis Example 1 except that 36.57 g of mesityl(3-(trifluoromethyl)phenyl)iodonium chloride was used instead of 36.76 g of bis(4-tert-butylphenyl)iodonium chloride as a raw material compound.
¹H-NMR (400 MHz, DMSO-D6): δ (ppm) = 2.31 (s, 12H), 2.61 (s, 24H), 7.25 (s, 8H), 7.73 (t, 4H), 8.01 (d, 4H), 8.15 (d, 4H), 8.43 (s, 4H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 6: tetrakis((5-fluoro-2-nitrophenyl)(2,4,6-trimethoxyphenyl)iodonium)silicotungstate (M-6)]

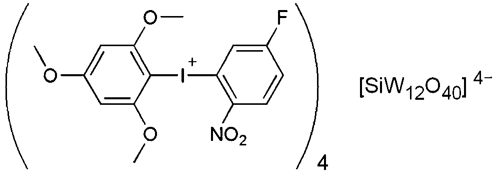

A target compound was produced in the same manner as in the salt exchange method of Synthesis Example 1 except that 40.27 g of (5-fluoro-2-nitrophenyl)(2,4,6-trimethoxyphenyl)iodonium chloride was used instead of 36.76 g of bis(4-tert-butylphenyl)iodonium chloride as a raw material compound.
¹H-NMR (400 MHz, DMSO-D6): δ (ppm) = 3.73 (s, 36H), 5.80 (s, 8H), 7.20 (m, 8H), 8.20 (d, 4H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 7: tetrakis((4-isobutylphenyl)(p-tolyl)iodonium)silicotungstate (M-7)]

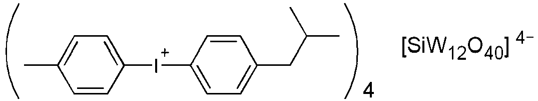

A target compound was produced in the same manner as in the salt exchange method of Synthesis Example 1 except that 33.15 g of (4-isobutylphenyl)(p-tolyl)iodonium chloride was used instead of 36.76 g of bis(4-tert-butylphenyl)iodonium chloride as a raw material compound.
¹H-NMR (400 MHz, DMSO-D6): δ (ppm) = 1.01 (d, 24H), 2.22 (m, 4H), 2.35 (s, 12H), 2.51 (d, 8H), 7.10-7.20 (m, 32H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 8: tetrakis((4-((2-hydroxytetradecyl)oxy)phenyl)(phenyl)iodonium)silicotungstate (M-8)]

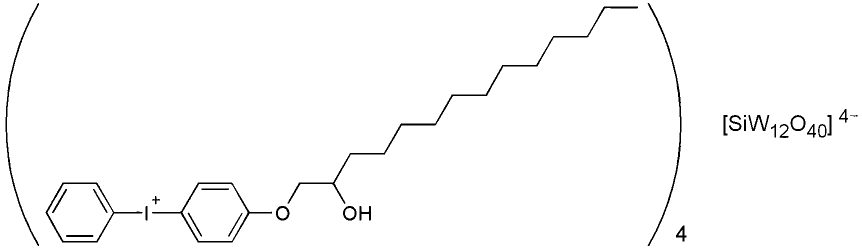

A target compound was produced in the same manner as in the salt exchange method of Synthesis Example 1 except that 46.72 g of (4-((2-hydroxytetradecyl)oxy)phenyl)(phenyl)iodonium chloride was used instead of 36.76 g of bis(4-tert-butylphenyl)iodonium chloride as a raw material compound.
¹H-NMR (400 MHz, DMSO-D6): δ (ppm) = 0.85 (t, 12H), 1.23-1.47 (m, 88H), 3.69-3.76 (m, 4H), 3.83-3.91 (m, 8H), 4.84 (d, 4H), 7.07 (d, 8H), 7.52 (t, 8H), 7.62-7.66 (m, 4H), 8.16-8.22 (m, 16H).
¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 9: tetrakis(diphenyliodonium)silicotungstate (M-9)]

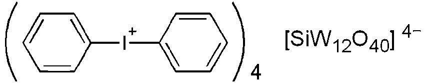

A target compound was produced in the same manner as in the salt exchange method of Synthesis Example 1 except that 27.13 g of diphenyliodonium chloride was used instead of 36.76 g of bis(4-tert-butylphenyl)iodonium chloride as a raw material compound.
¹H-NMR (400 MHz, DMSO-D6): δ (ppm) = 8.02 (d, 16H), 7.59 (t, 8H), 7.41 (t, 16H). ¹⁸³W-NMR (20.84 MHz, DMSO-D6): δ (ppm) = -92.7 (s, 12W).
ESI-MS: NEGATIVE m/z 718.5 (median) ([SiW₁₂O₄₀]⁴⁻)
XPS analysis I:W (the mole ratio of I to W) = 4:12

### [Synthesis Example 10: tris(diphenyliodonium)phosphotungstate (M-10)]

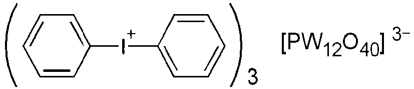

A target compound was produced in the same manner as in the salt exchange method of Synthesis Example 1 except that, as raw material compounds, 20.36 g of diphenyliodonium chloride was used instead of 36.76 g of bis(4-tert-butylphenyl)iodonium chloride, and 41.97 g of phosphotungstic acid (H₃[PW₁₂O₄₀]·30H₂O) was used instead of silicotungstic acid.
¹H-NMR (400 MHz, DMSO-D6): δ (ppm) = 8.02 (d, 12H), 7.59 (t, 8H), 7.41 (t, 12H).
¹⁸³W-NMR (20.84 MHz, DMSO-D6): δ (ppm) = -86.7 (s, 12W).
ESI-MS: NEGATIVE m/z 959 (median) ([PW₁₂O₄₀]³⁻)
XPS analysis I:W (the mole ratio of I to W) = 3:12

### [Test Example: Evaluation of Solubility in Solvent]

For the heteropolyoxotungstate compounds M-1 to M-10 produced in Synthesis Examples 1 to 10, the solubility in a solvent (methanol, acetonitrile, or dimethylformamide) was tested by the following method.

1 g of each of M-1 to M-10 powders was weighed in a beaker, the solvent was added thereto, and the weight concentration (% by weight) of each of M-1 to M-10 was calculated from the amount of the solvent added at the time when dissolution was visually confirmed, and was evaluated according to the following criteria. Table 1 shows the results.
Excellent: dissolved at 10% or more by weight
Good: dissolved at 5% or more by weight and less than 10% by weight
Fair: dissolved at 1% or more by weight and less than 5% by weight
Poor: not dissolved at less than 1% by weight

**[Table 1]**

| Compound | Solubility in a solvent | | |
|---|---|---|---|
| | Methanol | Acetonitrile | Dimethylformamide |
| M-1 | Good | Good | Excellent |
| M-2 | Good | Good | Excellent |
| M-3 | Fair | Good | Excellent |
| M-4 | Poor | Fair | Good |
| M-5 | Fair | Fair | Excellent |
| M-6 | Fair | Fair | Good |
| M-7 | Fair | Fair | Excellent |
| M-8 | Fair | Fair | Excellent |
| M-9 | Poor | Poor | Fair |
| M-10 | Poor | Poor | Fair |

Table 1 shows that, as in M-1 and M-2, in a case where the iodonium cation has a substituent recognized to be fat-soluble, such as a t-butyl group, on a benzene ring bonded to the iodine atom, the solubility in methanol and acetonitrile tends to be good, and particularly the solubility in dimethylformamide tends to be very good. On the other hand, as in M-9 and M-10, in a case where the iodonium cation has two identical benzene rings bonded to the iodine atom or has identical substituents on the benzene rings, that is, has a symmetrical structure, and has no substituent, the solubility in dimethylformamide is very low, and the possibility of use as a functional molecular block is recognized. On the other hand, the results showed that M-9 and M-10 had lower solubility in methanol and acetonitrile than M-1 and M-2.

As in M-3, in a case where the iodonium cation had two mesityl groups bonded to the iodine atom, the solubility in methanol was fair, the solubility in acetonitrile was good, and the solubility in dimethylformamide was very good.

As in M-4, in a case where the iodonium cation had a nitro group, which is an electron-withdrawing group, introduced on one benzene ring bonded to the iodine atom, the solubility in methanol was poor probably due to the occurrence of orientation by electronic polarization. On the other hand, the solubility in acetonitrile was fair, and the solubility in dimethylformamide was good.

As in M-5, in a case where the iodonium cation had a mesityl group bonded to one side of the iodine atom and a benzene ring substituted by a trifluoromethyl group, which is an electron-withdrawing group, bonded to the other side, the solubility in methanol and acetonitrile was fair and the solubility in dimethylformamide was very good possibly because the electronic polarization was reduced as compared with M-4.

As in M-6, in a case where the iodonium cation had a benzene ring with a methoxy group, which is an electron-donating group, bonded to one side of the iodine atom and a benzene ring with a nitro group and a fluorine atom, which are electron-withdrawing groups, bonded to the other side, the solubility in methanol and acetonitrile was fair, and the solubility in dimethylformamide was good.

As in M-7 and M-8, in a case where the iodonium cation had a benzene ring substituted by an isobutyl group or by a long-chain alkyl group on one side of the iodine atom, the solubility in each solvent changed little, the solubility in methanol and acetonitrile was fair, and the solubility in dimethylformamide was very good.

A heteropolyoxotungstate compound with high solubility in a solvent can be dissolved in the solvent and form a uniform film or the like, and can be suitably used as a catalyst with a redox ability, a magnetic material, or an actinic-ray-sensitive or radiation-sensitive compound.

## Claims

1. A heteropolyoxotungstate compound represented by the general formula (I) or a solvate thereof:
(A^{m+})_{x/m}(Bⁿ⁺)_{y/n}(C^{-(x+y)}) (I)
wherein
A^{m+} each independently denotes H⁺, a metal ion, or an ammonium ion,
Bⁿ⁺ each independently denotes an iodonium cation,
C^{-(x+y)} denotes a heteropolyoxotungstic acid, and
m denotes an integer in the range of 1 to 5, n denotes an integer of 1 or 2, x and x/m denote an integer, and y and y/n denote a natural number.

2. The heteropolyoxotungstate compound or the solvate thereof according to Claim 1, wherein the heteropolyoxotungstic acid is a heteropolyoxotungstic acid represented by the general formula (II-1) or (II-2), or a heteropolyoxotungstic acid represented as a deficient species thereof:
[XW₁₂O₄₀]^{-z1} (II-1)
[X₂W₁₈O₆₂]^{-z2} (II-2)
wherein
X each independently denotes B, Al, Si^{IV}, Ge^{IV}, P^{V}, As^{V}, V^{V}, Cr^{III}, Fe^{III}, Co^{III}, Co^{II}, Cu^{II}, Cu^{I}, Zn, Se^{VI}, or S^{VI}, and
z1 and z2 denote a natural number.

3. The heteropolyoxotungstate compound or the solvate thereof according to Claim 2, wherein the heteropolyoxotungstic acid represented by (II-1) is [PW₁₂O₄₀]³⁻, [SiW₁₂O₄₀]⁴⁻, [BW₁₂O₄₀]⁵⁻, or [SW₁₂O₄₀]²⁻.

4. The heteropolyoxotungstate compound or the solvate thereof according to Claim 2, wherein the heteropolyoxotungstic acid represented by (II-2) is [P₂W₁₈O₆₂]⁶⁻, [Si₂W₁₈O₆₂]⁸⁻, or [S₂W₁₈O₆₂]⁴⁻.

5. A heteropolyoxotungstate mixture comprising two or more different heteropolyoxotungstate compounds or solvates thereof according to Claim 1.

6. A heteropolyoxotungstate mixture comprising two or more different heteropolyoxotungstate compounds or solvates thereof according to Claim 1, wherein the heteropolyoxotungstic acid is silicotungstic acid of Keggin type or Dawson type or a deficient species thereof, and a mole ratio I:W of I to W in XRF analysis or XPS analysis is 0.5 to 10:9 to 18.

7. A heteropolyoxotungstate mixture comprising two or more different heteropolyoxotungstate compounds or solvates thereof according to Claim 1, wherein the heteropolyoxotungstic acid is phosphotungstic acid of Keggin type or Dawson type or a deficient species thereof, and a mole ratio I:W of I to W in XRF analysis or XPS analysis is 0.5 to 6:9 to 18.

8. The heteropolyoxotungstate compound or the solvate thereof according to Claim 1, wherein the iodonium cation is an iodonium cation represented by the general formula (III): 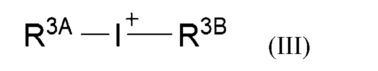 wherein
R^{3A} and R^{3B} each independently denote an optionally substituted C₁₋₁₈ hydrocarbyl group, an optionally substituted 3- to 18-membered non-aromatic heterocyclic group, or an optionally substituted 5- to 18-membered aromatic heterocyclic group;
R^{3A} and R^{3B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with an iodine atom in the formula (III).

9. The heteropolyoxotungstate compound or the solvate thereof according to Claim 1, wherein the iodonium cation is an iodonium cation represented by the general formula (IV-1): 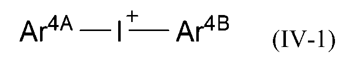 wherein
Ar^{4A} and Ar^{4B} each independently denote an optionally substituted C₆₋₁₈ aryl group or 5- to 18-membered heterocyclic group,
in the optionally substituted C₆₋₁₈ aryl group or 5- to 18-membered heterocyclic group, at least part of its hydrogen atoms may be substituted by a halogen atom, a hydroxy group, a thiol group, a nitro group, a cyano group, a carboxy group, an amino group, a sulfo group, or at least part of its hydrogen atoms may be substituted by a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, each of which may be substituted by a halogen atom, a hydroxy group, a thiol group, a nitro group, a cyano group, a carboxy group, an amino group, a sulfo group, or a group in which a hydrogen atom of a carboxy group or a hydroxy group is substituted by an acid-dissociable group,
a divalent carbon atom at any position excluding the terminals of the substituent may be replaced by -O-, -C(=O)-, -COO-, -OCO-, -CONH-, -NHCO-, -S-, or -SO₂-(provided that adjacent divalent carbon atoms are not replaced at the same time),
Ar^{4A} and Ar^{4B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with an iodine atom in the formula (IV-1); or
an iodonium cation represented by the general formula (IV-2): 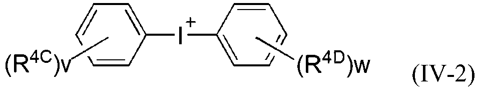 wherein
R^{4C} and R^{4D} each independently denote a halogen atom, a hydroxy group, a thiol group, a nitro group, a cyano group, a carboxy group, an amino group, a sulfo group, or each independently denote a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of its hydrogen atoms may be substituted by a halogen atom, a hydroxy group, a thiol group, a nitro group, a cyano group, a carboxy group, an amino group, or a sulfo group,
a divalent carbon atom at any position excluding the terminals of these may be replaced by -O-, -C(=O)-, -COO-, -OCO-, -CONH-, -NHCO-, -S-, or -SO₂- (provided that adjacent divalent carbon atoms are not replaced at the same time),
v and w each independently denote an integer in the range of 0 to 5, and when v to w denote an integer of 2 or more, R^{4A} and R^{4D} may be the same or different.

10. The heteropolyoxotungstate compound or the solvate thereof according to Claim 8, wherein in an iodonium cation represented by the general formula (IV-1), Ar^{4B} denotes an iodonium cation represented by the general formula (IV-3): 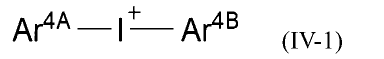 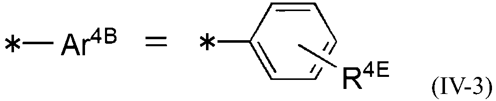 wherein
R^{4E} denotes a halogen atom, a nitro group, a C₁₋₄ haloalkyl group, or an optionally substituted branched C₃₋₁₀ alkyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group, and
when R^{4E} denotes a halogen atom, a nitro group, or a C₁₋₄ haloalkyl group, Ar^{4A} and Ar^{4B} are different from each other.

11. The heteropolyoxotungstate compound or the solvate thereof according to Claim 8, wherein in an iodonium cation represented by the general formula (IV-1), Ar^{4B} denotes an iodonium cation represented by the general formula (IV-4): 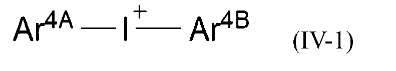 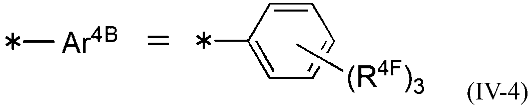 wherein
R^{4F} each independently denotes an optionally substituted C₁₋₁₂ hydrocarbyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group.

12. A method for producing a heteropolyoxotungstate compound represented by the general formula (I) or a solvate thereof, the method comprising:
reacting a compound represented by the general formula (V-1) with a compound represented by the general formula (V-2) to perform salt exchange;
(A^{m+})_{x/m}(Bⁿ⁺)_{y/n}(C^{-(x+y)}) (I)
(A^{m+})_{x/m}(A'^{m'+})_{y/m'}(C^{-(x+y)}) (V1)
(Bⁿ⁺)_{y/n}(Y⁻)_{y} (V-2)
wherein
A^{m+} each independently denotes H⁺, a metal ion, or an ammonium ion;
A'^{m'+} each independently denotes a metal ion or an ammonium ion;
Bⁿ⁺ each independently denotes an iodonium cation;
C^{-(x+y)} denotes a heteropolyoxotungstic acid;
Y⁻ denotes a monovalent counter anion; and
m and m' denote an integer in the range of 1 to 5, n denotes an integer of 1 or 2, x and x/m denote an integer, and y/m', y, and y/n denote a natural number.

13. A method for producing a heteropolyoxotungstate compound represented by the general formula (I) or a solvate thereof, the method comprising:
reacting a compound represented by the general formula (V-1) with a compound represented by the general formula (V-2) to perform salt exchange; and
removing a compound represented by the general formula (V-3) from the resulting compounds represented by the general formulae (I) and (V-3);
(A^{m+})_{x/m}(Bⁿ⁺)_{y/n}(C^{-(x+y)}) (I)
(A^{m+})_{x/m}(A'^{m'+})_{y/m}(C^{-(x+y)}) (V1)
(Bⁿ⁺)_{y/n}(Y⁻)_{y} (V-2)
(A'^{m'+})_{y/m'}(Y⁻)_{y} (V-3)
wherein
A^{m+} each independently denotes H⁺, a metal ion, or an ammonium ion;
A'^{m'+} each independently denotes a metal ion or an ammonium ion;
Bⁿ⁺ each independently denotes an iodonium cation;
C^{-(x+y)} denotes a heteropolyoxotungstic acid;
Y⁻ denotes a monovalent counter anion; and
m and m' denote an integer in the range of 1 to 5, n denotes an integer of 1 or 2, x and x/m denote an integer, and y/m', y, and y/n denote a natural number.

14. A method for producing a heteropolyoxotungstate compound represented by the general formula (I) or a solvate thereof, the method comprising:
ion-exchanging A'^{m'+} of a compound represented by the general formula (V-1) with H⁺ using an ion-exchange resin; and
reacting the resulting compound represented by the general formula (V-4) with a compound represented by the general formula (V-2);
(A^{m+})_{x/m}(Bⁿ⁺)_{y/n}(C^{-(x+y)}) (I)
(A^{m+})_{x/m}(A'^{m'+})_{y/m'}(C^{-(x+y)}) (V1)
(A^{m+})_{x/m}(H⁺)_{y}(C^{-(x+y)}) (V-4)
(Bⁿ⁺)_{y/n}(Y⁻)_{y} (V-2)
wherein
A^{m+} each independently denotes H⁺, a metal ion, or an ammonium ion;
A'^{m'+} each independently denotes a metal ion or an ammonium ion;
Bⁿ⁺ each independently denotes an iodonium cation;
C^{-(x+y)} denotes a heteropolyoxotungstic acid;
Y⁻ denotes a monovalent counter anion; and
m and m' denote an integer in the range of 1 to 5, n denotes an integer of 1 or 2, x and x/m denote an integer, and y/m', y, and y/n denote a natural number.
